(19) Europäisches Patentamt
European Patent Office
Office européen des brevets

(11) **EP 1 568 996 B1**

(12) **EUROPÄISCHE PATENTSCHRIFT**

(45) Veröffentlichungstag und Bekanntmachung des Hinweises auf die Patenterteilung:
**20.08.2014 Patentblatt 2014/34**

(51) Int Cl.:
*G01N 33/543* (2006.01)     *G01N 33/58* (2006.01)
*G01N 33/569* (2006.01)     *G01N 33/576* (2006.01)
*G01N 33/545* (2006.01)

(21) Anmeldenummer: **05008770.9**

(22) Anmeldetag: **22.06.1999**

(54) **Verbesserung von Bindeassays durch Multiepitopanalyse und Kombination von Antigen- und Antikörper- Bestimmung**

Improved binding assays through multiepitope analysis and combined antigen and antibody determination

Amélioration d'essais de liaison par analyse au moyen d'épitopes multiples et combinaison de la détermination d'un antigène et d'un anticorps

(84) Benannte Vertragsstaaten:
**AT CH DE ES FR GB IT LI NL**

(30) Priorität: **22.06.1998 DE 19827714**
**26.08.1998 DE 19838802**

(43) Veröffentlichungstag der Anmeldung:
**31.08.2005 Patentblatt 2005/35**

(62) Dokumentnummer(n) der früheren Anmeldung(en) nach Art. 76 EPÜ:
**99931132.7 / 1 090 298**

(73) Patentinhaber: **Roche Diagnostics GmbH**
**68305 Mannheim (DE)**

(72) Erfinder:
• **Karl, Johann, Dr.**
**82380 Peissenberg (DE)**
• **Hornauer, Hans, Dr.**
**82380 Peissenberg (DE)**

(74) Vertreter: **Dey, Michael et al**
**Weickmann & Weickmann**
**Patentanwälte**
**Richard-Strauss-Strasse 80**
**81679 München (DE)**

(56) Entgegenhaltungen:
**EP-A- 0 461 462     WO-A-93/08472**
**WO-A-97/32212     US-A- 5 627 026**

• **R. EKINS ET AL.: "Multianalyte microspot imunoassay. The microanalytical "compact disk" of the future." ANNALES DE BIOLOGIE CLINIQUE, Bd. 50, Nr. 5, 1992, Seiten 337-353, XP000617908 PARIS, FR.**
• **R. EKINS ET AL.: "Development of microspot multi-analyte ratiometric immunoassay using dual fluorescent--labelled antibodies." ANALYTICA CHIMICA ACTA, Bd. 227, Nr. 1, 1. Dezember 1989 (1989-12-01), Seiten 73-96, XP002108690 AMSTERDAM, NL ISSN: 0003-2670**
• **S. E. KAKABAKOS ET AL.: "Multianalyte immunoassay based on spatially distinct fluorescent areas quantified by laser-excited solid-phase time-resolved fluorometry" CLINICAL CHEMISTRY., Bd. 38, Nr. 3, März 1992 (1992-03), Seiten 338-342, XP002108659 AMERICAN ASSOCIATION FOR CLINICAL CHEMISTRY. WINSTON., US ISSN: 0009-9147**

**Beschreibung**

Offenbart wird ein Verfahren zum Nachweis eines oder mehrerer Analyten in einer Probe, wobei der Nachweis des Analyten mit unterschiedlichen, an den Analyten bindefähigen Reagenzien geführt wird. Weiterhin offenbart wird eine Festphase zum Nachweis eines Analyten, wobei die Festphase einen nichtporösen Träger und räumlich getrennte Testflächen umfaßt, wobei jede Testfläche unterschiedliche Reagenzien enthält. Die Erfindung betrifft ein Verfahren zur gleichzeitigen Bestimmung eines Antigens und eines spezifisch gegen dieses Antigen gerichteten Antikörpers sowie die Verwendung einer Festphase zur Durchführung dieses Verfahrens.

Eine Vielzahl von Analyten kann durch immunologische Nachweisverfahren bestimmt werden. Solche immunologische Nachweisverfahren nutzen die spezifische Bindefähigkeit von Analyten mit bestimmten Reagenzien, wie beispielsweise Antigen-Antikörper-Wechselwirkungen. Grundsätzlich können immunologische Bestimmungen in einer Reihe von Testformaten durchgeführt werden, wie etwa dem Sandwichtestformat, dem indirekten Testformat, dem Rücktitrationsformat oder dem Brückenformat.

Ein zuverlässiger Nachweis von Infektionserkrankungen, z.B. einer Infizierung mit Viren wie etwa HIV, HBV oder HCV, ist von besonderem Interesse, um die Erkrankung bei betroffenen Personen möglichst frühzeitig diagnostizieren zu können. Im Allgemeinen werden immunologische Bestimmungen von Antikörpern gegen HIV, HBV oder HCV im indirekten Testformat oder mittels Brückenformat durchgeführt. Zum Nachweis der Antikörper werden dabei zumeist Gemische verschiedener Proteine bzw. Peptide verwendet, die Epitope aus der Core- und Envelope-Region des Erregers umfassen. Dieses Gemisch wird auf einem Träger, d.h. einer Festphase, immobilisiert. Da die Einstufung als HIV-positiv für den Einzelnen von großer Bedeutung ist und falsch positive Ergebnisse fatale Folgen haben können, ist es derzeit notwendig, alle bei dieser immunologischen Bestimmung in Routinetests erhaltenen positiven Ergebnisse in einem Bestätigungstest zu überprüfen. Als Bestätigungstest wird üblicherweise ein Westernblot verwendet, bei dem die einzelnen Proteinbestandteile eines Viruslysats auf einen porösen Träger aufgeblottet sind. Im Fall von HCV ist es allerdings sehr schwierig, den Virus zu züchten. Deshalb wird hier als Bestätigungstest kein Westernblot mit Viruslysat sondern ein RIBA (Recombinant Immunoblot Assay) durchgeführt, bei dem es sich um einen Immunodotblot, der rekombinante Proteine bzw. Peptide als Testreagenzien umfaßt, handelt.

Ein großer Nachteil der derzeit verwendeten Routinetests besteht darin, daß je nach Analyt Gemische von 5 bis 10 oder mehr Antigenen zum Nachweis eingesetzt werden. Zwar werden die Routinetests laufend verbessert, ein vollständiger Verzicht auf Bestätigungstests konnte jedoch noch nicht erreicht werden. Beispielsweise wird im Enzymun® HIV-Test (Boehringer Mannheim) ein Gemisch aus ca. 5 verschiedenen Antigenen verwendet, welche für den Nachweis sowohl biotinyliert als auch Digoxigenin-markiert sind. Obwohl der Test gut funktioniert, bedeutet die Verwendung von Antigengemischen mit einer derart hohen Anzahl verschiedener Antigene, daß die einzelnen auf der Festphase immobilisierten bzw. gebundenen Antigene nicht mehr in einer für den Nachweis optimalen Konzentration vorliegen können. Die Bindekapazität der Festphase ist bei einem solchen Gemisch aus einer Vielzahl von Komponenten nicht mehr ausreichend, um alle Antigene in der optimalen Konzentration zu binden. Weiterhin hat die Verwendung eines Antigen-Gemisches bei der Beschichtung einer Testfläche zur Folge, daß die verschiedenen Antigene um die Bindungsstellen auf der Festphase kompetieren und die unterschiedlichen Größenverhältnisse zu unterschiedlichen Diffusionsgeschwindigkeiten und zu unterschiedlichen sterischen Effekten führen. Bei einer Direktbeschichtung werden z.B. hydrophobe Antigene bevorzugt an die Kunststoffoberfläche gebunden, während gleichzeitig hydrophilere Antigene verdrängt werden. Dies führt dazu, daß zum einen nur schlecht reproduzierbare Ergebnisse erhalten werden und zum anderen die Konzentration bestimmter Antigen-Epitope so gering wird, daß ein signifikanter Nachweis nicht mehr möglich ist.

Ein weiterer Nachteil der Verwendung von Antigengemischen in Routinetests liegt darin, daß durch das Gemisch unterschiedlicher Antigene die Gefahr einer erhöhten unspezifischen Bindung deutlich gesteigert wird, was wiederum zu einem Anstieg falsch positiver Ergebnisse führt. Dies bewirkt, daß bei den bisher verwendeten Routinetests die Cut-off-Grenze relativ hoch gesetzt werden muß und somit Sensitivität verloren geht. Vor allem im Westernblot nimmt aufgrund von im Viruslysat vorliegenden Fremdproteinen die Zahl der falschpositiven Ergebnisse aufgrund unspezifischer Bindung deutlich zu, so daß mindestens 2 reaktive Banden für ein positives Ergebnis gefordert werden.

Es wurde versucht, die Sensitivität dieser Nachweisverfahren weiter zu verbessern. EP 0 461 462 A1 beschreibt einen Immunoassay zum Nachweis von viralen Antikörpern mit Hilfe eines indirekten Testkonzepts. Bei dem in EP 0 461 462 A1 beschriebenen Immunodotblot werden anstelle eines üblichen Viruslysats aufgereinigte rekombinante Proteine einzeln in diskreten Testflächen auf einen porösen Träger aufgetragen, wobei ein Testformat erhalten wird, das aufgrund der Verwendung aufgereinigter Proteine sensitiver als ein Westernblot ist.

EP 0 627 625 A1 betrifft ein Verfahren zum Nachweis von viralen Antikörpern in einer Probe mittels Brückenkonzept. Auch bei diesem Verfahren handelt es sich um einen RIBA (Recombinant Immunoblot Assay), bei dem mehrere Antigene räumlich getrennt auf eine Festphase aus einem porösen Material aufgebracht werden, wobei auf die Notwendigkeit der Verwendung einer Festphase aus porösem Material hingewiesen wird.

EP 0 445 423 A2 betrifft ein Verfahren zum Nachweis von HCV-Antikörpern mit Hilfe mehrerer Epitope eines HCV-Antigens. Auch in EP 0 445 423 A2 wird ein Immunodotassay zur Antikörperbestimmung beschrieben, wobei eine höhere Sensitivität durch die Verwendung bestimmter, verbesserter Antigene erzielt wird.

Bei diesen im Stand der Technik beschriebenen Verfahren ist jedoch aufgrund der Verwendung eines porösen Trägers das definierte Aufbringen einer vorbestimmten Reagenzmenge schwierig. Insbesondere besteht die Gefahr des Ineinanderlaufens der einzelnen aufgebrachten Testspots. Diese Nachteile werden um so gravierender, je kleiner die aufgebrachten Spots werden, so daß diese Verfahren insbesondere für miniaturisierte Testsysteme nicht geeignet sind. Außerdem ist die Handhabung von Papierstreifen schwer automatisierbar und somit als Routinetest nicht denkbar.

Eine Aufgabe bestand deshalb darin, ein Verfahren bereitzustellen, durch das die im Stand der Technik auftretenden Nachteile zumindest teilweise beseitigt werden können.

Diese Aufgabe wird gelöst durch ein Verfahren zum Nachweis eines Analyten in einer Probe, umfassend die Schritte:

(a) Bereitstellen einer Festphase, die einen nichtporösen Träger und mindestens zwei räumlich getrennte Testflächen umfaßt, wobei die Testflächen jeweils unterschiedliche, immobilisierte analytspezifische Rezeptoren enthalten,
(b) Inkontaktbringen der Probe mit der Festphase und mindestens einem freien analytspezifischen Rezeptor, der eine signalgebende Gruppe trägt oder mit einer signalgebenden Gruppe bindefähig ist, und
(c) Nachweisen des Vorhandenseins oder/und der Menge des Analyten durch Bestimmung der signalgebenden Gruppe auf den Testflächen.

Der immobilisierte analytspezifische Rezeptor kann sowohl direkt als auch indirekt über einen oder mehrere Rezeptoren an die Festphase gebunden sein. Die Bindung kann beispielsweise durch adsorptive oder kovalente Wechselwirkungen, bevorzugt jedoch durch spezifische hochaffine Wechselwirkungen, z.B. Streptavidin oder Avidin/Biotin oder Antikörper-Antigen-Wechselwirkungen erfolgen.

Der freie analytspezifische Rezeptor kann selbst eine signalgebende Gruppe tragen oder mit einer signalgebenden Gruppe bindefähig sein. In diesem Fall besteht das Nachweisreagenz aus mehreren Komponenten.

Bei dem Analyten kann es sich um eine homogene oder um eine heterogene Population, z.B. eine heterogene Antikörperpopulation, ein Antigengemisch oder ein Gemisch von gegebenenfalls unterschiedlichen Antigenen und Antikörpern handeln, wobei die Antigene und Antikörper von einem oder mehreren Erregern stammen bzw. induziert werden. Die einzelnen Testflächen binden im Fall von heterogenen Analytpopulationen eine Teilpopulation des zu bestimmenden Analyten. Die jeweils auf einer Testfläche immobilisierten analytspezifischen Rezeptoren sind unterschiedlich, d.h. sie binden erfindungsgemäß vorzugsweise an verschiedene Epitope eines homogenen Analyten wie etwa eines Antigens, an verschiedene Analytsubtypen wie etwa Antigensubtypen oder/und an verschiedene Analyttypen wie etwa unterschiedliche Antigene oder/und Antikörper.

Überraschenderweise wurde festgestellt, daß die Sensitivität von Nachweistests, wie etwa Antikörpertests, durch die Verwendung von Paneltests, bei denen die verschiedenen Reagenzien, z.B. verschiedene Antigene, als Einzelspots, d.h. einzeln auf separaten Testflächen, aufgetragen werden, deutlich verbessert werden kann. Durch die erfindungsgemäße Multiepitopanalyse, d.h. den gleichzeitigen separaten Nachweis mehrerer Teilpopulationen eines Analyten oder Erregers, wie etwa HIV, kann die Sensitivität und Zuverlässigkeit von Nachweistests erheblich gesteigert werden.

Wenn auf einer oder mehreren, in manchen Fällen auf mindestens zwei, Testflächen ein positives Testergebnis erhalten wird, wird dies als Vorhandensein des Analyten in der Probe gewertet.

Durch die Verwendung eines nichtporösen Trägers, ist es möglich, die Reagenzien in definierten Flächen aufzubringen. Dies ist insbesondere bei miniaturisierten Testformaten von Bedeutung. Entsprechend weisen die Testflächen bevorzugt einen Durchmesser von 0,01 bis 1 mm, mehr bevorzugt von 0,1 bis 0,5 mm umd am meisten bevorzugt von 0,1 bis 0,2 mm auf.

Bevorzugt werden Festphasen mit mehreren Testflächen verwendet, die auch als Array-Systeme bezeichnet werden. Solche Array-Systeme sind z.B. bei Ekins und Chu (Clin. Chem. 37 (1995) 1955-1967) und in den US-Patenten 5,432,099, 5,516,635 und 5,126,276 beschrieben.

Die verwendete Festphase umfaßt einen für Nachweisverfahren verwendbaren, nichtporösen Träger. Der nichtporöse Träger kann dabei aus einem beliebigen, nichtporösen Material bestehen. Bevorzugt umfaßt der Träger eine Kunststoff-, Glas-, Metall- oder Metalloxidoberfläche. Besonders bevorzugt weist der Träger eine Polystyroloberfläche auf. Auf diesem Träger sind räumlich diskrete Bereiche (Testflächen) angeordnet. Auf diesen Testflächen sind Reagenzien, wie etwa immobilisierte Festphasenrezeptoren aufgebracht. Die Immobilisierung der Reagenzien auf den Testflächen erfolgt nach bekannten Methoden, z.B. durch direkte adsorptive Bindung, durch kovalente Kopplung oder durch Kopplung über hochaffine Bindepaare, z.B. Streptavidin/Biotin, Antigen/Antikörper oder Zucker/Lectin.

Besonders vorteilhaft ist, wenn die räumlich getrennten Testflächen separat mit verschiedenen Reagenzien beladen werden. Durch die Einzelauftragung der verschiedenen Testflächen können für jedes Reagenz, beispielsweise für jedes einzelne Antigen, die optimale Festphasenkonzentration und die optimalen Beschichtungsbedingungen z.B. in Form von speziellen Pufferrezepturen gewählt werden. Dadurch ist es möglich, jeden einzelnen analytspezifischen Rezeptor, z.B. jedes einzelne Antigen bis zur maximalen Bindekapazität der Fläche zu beschichten, während bei den bisher bekannten Tests jeder Rezeptor, z. B. jedes Antigen nur zu einem Teil der verfügbaren Bindekapazität gebunden werden konnte. Durch die separate Auftragung der verschiedenen Reagenzien findet außerdem keine Kompetition der einzelnen Reagenzien, beispielsweise der Antigene, um die Bindungsplätze auf der Festphase statt. Entsprechend ist es bevorzugt, jeweils nur ein Reagenz, das spezifisch mit dem zu bestimmenden Analyten bindefähig ist, pro Testfläche zu binden, so daß jede Testfläche nur einen einzigen Typ eines immobilisierten analytspezifischen Rezeptors enthält. Dieses Reagenz kann gegebenenfalls durch inerte Verdünnermoleküle verdünnt werden, um eine optimale homogene Bindephase zu bilden. Inerte Verdünnermoleküle sind Moleküle, die an die Festphase binden aber keine Wechselwirkung mit dem Analyten oder anderen Probebestandteilen eingehen. Geeignete Verdünnermoleküle sind beispielsweise in WO 92/10757 und in EP 0 664 452 A2 beschrieben.

Bei Testflächen, auf denen nur ein einziges, mit dem Analyten bindefähiges Reagenz, wie etwa ein Antigen, gebunden ist, wurde festgestellt, daß die unspezifische Bindung deutlich reduziert ist. So ist z.B. bei Auftragung verschiedener Antigene als Einzelspots keine meßbare unspezifische Bindung zu beobachten, während ein Testspot, auf den ein Gemisch aus mehreren Antigenen aufgebracht wurde, eine deutlich meßbare unspezifische Bindung zeigt.

Der Nachweis des Analyten erfolgt im Verfahren auf bekannte Weise durch Verwendung geeigneter Markierungsgruppen, z.B. Fluoreszenzmarkierungsgruppen, chemilumineszierender Gruppen, radioaktiven Markierungen, Enzymmarkierungen, farbigen Markierungen und Solpartikeln. Alternativ kann, bei geeigneten Festphasen, die Wechselwirkung von Bestandteilen des Nachweismediums mit den Testflächen auch durch Bestimmung der Schichtdicke der jeweiligen Flächen z.B. durch Plasmonenresonanzspektroskopie, nachgewiesen werden.

Die begrenzten Testflächen können darüber hinaus zur Unterscheidung gegenüber inerten Bereichen der Festphase eine nachweisbare und analytunspezifische Markierungsgruppe enthalten, die neben der analytspezifischen Beschichtungsgruppe nachweisbar ist und mit ihr nicht interferiert. Ein Beispiel für eine solche analytunspezifische Markierungsgruppe ist eine Fluoreszenz-Markierungsgruppe, die bei einer Wellenlänge fluoresziert, die von der Fluoreszenzwellenlänge einer analytspezifischen Markierungsgruppe verschieden ist. Die analytunspezifische Markierungsgruppe wird vorzugsweise, ebenso wie der Festphasenrezeptor, überein hochaffines Bindepaar, z.B. Streptavidin/Biotin immobilisiert.

Eine weitere Sensitivitätssteigerung kann durch die Verwendung eines universellen Nachweisreagenz erreicht werden. Zuvor kann für jedes Analytmolekül ein separates Nachweisreagenz, welches an das Analytmolekül bindet und eine Markierung, wie etwa ein Enzym, einen Fluoreszenzmarker oder fluoreszierende Latexpartikel trägt, eingesetzt werden. Durch die Kombination mehrerer markierter Nachweisreagenzien wird jedoch oftmals die Konzentration an Markierungen sehr hoch, so daß die unspezifische Bindung naturgemäß stark zunimmt. Dieses Problem kann durch die Verwendung eines universellen Nachweisreagenz gelöst werden. Bevorzugt werden fluoreszenzmarkierte Latexpartikel als universelles Nachweisreagenz verwendet. Dabei wird für die spezifische Bindung des Analytmoleküls ein analytspezifischer erster Rezeptor verwendet, der selbt keine signalgebende Gruppe trägt. An diesen analytspezifischen ersten Rezeptor bindet ein universeller zweiter markierter Rezeptor, d.h. ein Rezeptor, der analytunabhängig an mehrere, vorzugsweise an alle verwendeten ersten Rezeptoren bindet. Die Kopplung des zweiten Rezeptors an die Markierungsgruppe kann adsorptiv, kovalent über funktionelle Gruppen erfolgen oder über hochaffine Bindepaare, z.B. Streptavidin/Biotin, Antigen/Antikörper oder Zucker/Lectin erfolgen. Bevorzugt wird das bekannte Dig/Anti-Dig-System verwendet.

Ein weiterer Nachteil der Brückentests, die z.B. als 1-Schritt-Reaktion ausgeführt werden, ist, daß der Festphasenrezeptor (z.B. biotinyliertes HIV-gp41) und der freie Nachweisrezeptor (z.B. digoxigenyliertes HIV-gp41) im Verhältnis 1:1 angeboten werden müssen, um ein optimales Signal zu erzielen. Dies ist nachteilig, da aufgrund der beschränkten Bindekapazität der Festphase die Konzentration der einzelnen Festphasenrezeptoren oftmals suboptimal ist und somit auch für den Nachweisrezeptor nicht günstig liegen kann.

Mit dem Verfahren können die Festphasenrezeptoren bereits auf der Festphase in optimaler Konzentration gebunden werden. Weiterhin kann auch der Nachweisrezeptor in optimaler Konzentration angeboten werden, da Rezeptorkonjugate mit Digoxigenin oder Biotin im Gegensatz zu Enzym-markierten Rezeptoren nur unwesentlich zu unspezifischer Bindung neigen. Man kann diese Reagenzien im Überschuß einsetzen, so daß Impräzisionen bei der Zugabe des Rezeptors nicht auf die Testpräzision durchschlagen.

Durch spezifische Bindung des zu bestimmenden Analyten an das auf die Testfläche immobilisierte Reagenz, z.B. einen Festphasenrezeptor, kann das Vorhandensein oder/und die Menge des Analyten in einer Probe bestimmt werden. Durch kombinierte Auswertung der verschiedenen Testflächen, die jeweils unterschiedliche, spezifisch mit dem Analyten bindefähige Reagenzien enthalten, kann die Sensitivität des Nachweisverfahrens, insbesondere durch eine Verringerung falsch positiver Ergebnisse und die zweifelsfreie Erkennung richtig positiver Ergebnisse, merklich verbessert werden. Von besonderem Interesse ist das Verfahren zur Erfassung bzw. Eliminierung von unspezifischen Bindungen bei qualitativen Tests mit hohen Anforderungen an die Spezifität, wie etwa bei Tests auf Infektionen (z.B. HIV).

Durch die Verwendung von Arrays, d.h. Festphasen, die mindestens zwei, mehr bevorzugt mindestens drei, am meisten bevorzugt mindestest fünf, und bis zu eintausend, mehr bevorzugt bis zu einhundert räumlich getrennte Testflächen umfassen, ist es möglich, mindestens eine dieser Testflächen so auszugestalten, daß sie eine Kontrollfläche darstellt. Folglich umfaßt das Verfahren bevorzugt die Verwendung einer Festphase, die zusätzlich mindestens eine, mehr bevorzugt zwei und am meisten bevorzugt mindestens fünf Kontrollflächen umfaßt. Durch die Integration von Kontrollspots in die Festphase ist es möglich, falsche Resultate aufgrund von Störungen leicht und schnell zu erkennen. Neben den spezifischen Testflächen, ist es weiterhin möglich, einen probenspezifischen Untergrund zu messen und damit einen probenspezifischen Cut-off zu definieren. Die Verwendung eines Testarrays und die Verwendung von Kontrollspots ermöglicht es, die Cut-off-Grenze abzusenken. Der Cut-Off-Wert ist ein Grenzwert, der bei Testverfahren eingesetzt wird, um zwischen positiven und negativen Werten unterscheiden zu können. Ein solcher Cut-Off-Wert ist insbesondere bei Testverfahren, welche Infektionskrankheiten betreffen, von Bedeutung. Mit Hilfe des Verfahrens ist es möglich eine Positiv-/Negativ-Unterscheidung mit erheblich geringerer Fehlerwahrscheinlichkeit zu treffen.

Bei Verwendung von mehreren Testflächen, die jeweils zur Bestimmung unterschiedlicher Analytmoleküle vorgesehen sind, hat sich oftmals eine testflächenspezifische Definition des Cut-Off-Werts als geeignet erwiesen, um eine erhöhte Testspezifität (d.h. korrekte Unterscheidung zwischen positiven und negativen Werten) bei gleichbleibender Sensitivität zu erhalten.

Das Verfahren kann für beliebigen Nachweismethoden eingesetzt werden, z.B. für Immunoassays, Nukleinsäure-Hybridisierungsassays, Zucker-Lectin-Assays und ähnliche Verfahren. Das erfindungsgemäße Verfahren eignet sich auch grundsätzlich zum Nachweis beliebiger Analyten in einer Probe. Besonders bevorzugt erfolgt der Nachweis des Analyten über spezifische Wechselwirkungen mit einem oder mehreren mit dem Analyten bindefähigen Reagenzien, d.h. Rezeptoren, die bevorzugt aus Proteinen, Peptiden, Antikörpern, Antigenen, Haptenen und Nukleinsäuren ausgewählt werden.

Während ein wesentlicher Vorteil des Verfahrens zunächst darin besteht, die Sensitivität des Nachweises eines einzigen Analyten zu verbessern, können bei geeigneter Wahl der Testflächen auch mehrere Analyten gleichzeitig mit hoher Sensitivität bestimmt werden.

Offenbart wird eine Festphase zum Nachweis eines Analyten in einer Probe, welche dadurch gekennzeichnet ist, daß sie einen nichtporösen Träger und mindestens zwei räumlich getrennte Testflächen umfaßt, wobei die Testflächen jeweils unterschiedliche Reagenzien enthalten, die spezifisch mit dem zu bestimmenden Analyten bindefähig sind.

Bevorzugt enthalten die Testflächen jeweils unterschiedliche Reagenzien, die an verschiedene Epitope oder/und Subtypen eines Analyten oder/und an verschiedene Analyttypen binden.

Um eine möglichst große Anzahl von Testflächen auf einer Festphase unterzubringen, werden bevorzugt miniaturisierte Testformate verwendet. Der Abstand zwischen den einzelnen Testflächen wird so gewählt, daß ein Ineinanderlaufen der aufgebrachten Reagenzien nicht möglich ist. Üblicherweise reicht es hierfür aus, wenn die Ränder der Testflächen einen Abstand von 0,05 bis 5 mm aufweisen. Zwischen den Testflächen befindet sich bevorzugt eine inerte Oberfläche, die weder mit dem Analyten noch mit sonstigen Probebestandteilen bindefähig ist.

Die Festphase kann in beliebigen Nachweisverfahren eingesetzt werden, z.B. in Immunoassays, Nukleinsäure-Hybridisierungsassays, Zucker-Lectin-Assays und dergleichen. Bevorzugt wird sie in einem Immunoassay zum Nachweis von Antikörpern oder/und Antigenen verwendet.

Weiterhin umfaßt die Erfindung die Verwendung eines Testkits wie beansprucht, welches die Verwendung einer Festphase sowie markierte Nachweisreagenzien umfaßt. Markierte Nachweisreagenzien sind dem Fachmann bekannt und umfassen im allgemeinen eine Markierungsgruppe sowie eine spezifisch bindefähige Gruppe, die den Nachweis des Analyten ermöglicht. Geeignete Markierungsgruppen sind z.B. Fluoreszenz-, Chemilumineszenz-, Enzym-, radioaktive oder Partikel-(Sol)-Markierungsgruppen. Die spezifisch bindefähige Gruppe kann z.B. mit dem gebildeten Analytkomplex bindefähig sein oder bei kompetitiven Testformaten mit anderen Bestandteilen des Nachweissystems. Bevorzugt umfaßt der Testkit ein universelles Konjugat als Nachweisreagenz, insbesondere fluoreszenzmarkierte Latexpartikel, welches mit den für den Analyten spezifischen Nachweisrezeptoren bindefähig ist.

Ein weiteres Problem herkömmlicher Routinetests besteht darin, daß die gleichzeitige Bestimmung eines Antigens und eines spezifisch gegen dieses Antigen gerichteten Antikörpers in einer Messung nicht durchführbar ist. Aus diesem Grund wird bei sogenannten HIV-Kombinationstests beispielsweise eine Bestimmung des Antigens p24 und von Antikörpern gegen andere HIV-Antigene gleichzeitig durchgeführt. Bei einem solchen Test können dann lediglich Antikörper gegen andere HIV-Antigene, wie beispielsweise gp41 oder gp120 bestimmt werden, während die Bestimmung von Antikörpern gegen p24 nicht möglich ist.

US-PS-5, 627,026 beschreibt ein Verfahren zum Nachweis eines Antikörpers und eines Antigens in einer biologischen Probe. So wird beispielsweise ein Assay zur Bestimmung des FeLV-Antigens und des FIV-Antikörpers beschrieben. Auch gemäß dem Verfahren von US-PS-5,627,026 können bei der Bestimmung eines Antigens im gleichen Test lediglich Antikörper, die gegen andere Antigene gerichtet sind, nachgewiesen werden.

Eine weitere Aufgabe der vorliegenden Erfindung bestand deshalb darin, ein Verfahren zur gleichzeitigen Bestimmung eines Antigens und eines spezifisch gegen dieses Antigen gerichteten Antikörpers in einer Probe bereitzustellen. Diese Aufgabe wird gelöst durch ein Verfahren umfassend die Schritte

(a) Bereitstellen einer Festphase, auf der in einer ersten Testfläche ein mit dem zu bestimmenden Antigen bindefähiger immobilisierter Rezeptor und in einer räumlich davon getrennten zweiten Testfläche ein mit dem zu bestimmenden Antikörper bindefähige immobilisierter Rezeptor aufgebracht ist,

(b) Inkontaktbringen der Probe mit der Festphase und einem freien analytspezifischen Rezeptor, der eine signalgebende Gruppe trägt oder mit einer signalgebenden Gruppe bindefähig ist und

(c) Nachweisen des Vorhandenseins oder/und der Menge des Antigens und des Antikörpers durch Bestimmung der signalgebenden Gruppe auf der Festphase.

Der Nachweis des Antigens findet bevorzugt unter Verwendung eines Sandwichtests und der Nachweis des Antikörpers bevorzugt unter Verwendung eines Brückenkonzepts, eines Rücktitrationskonzepts oder eines indirekten Testformats statt.

Bevorzugt wird der Nachweis des Antikörpers unter Verwendung einer Rücktitration durchgeführt. Vorteilhaft ist dabei, daß bei gleichzeitiger Verwendung eines Sandwichtests zum Nachweis des Antigens keine gegenseitige Beeinflussung von Nachweismolekülen stattfinden kann, da in diesem Fall für den Nachweis des Antigens und den Nachweis des Antikörpers das gleiche Nachweisreagenz verwendet werden kann. Für einen Sandwichtest zum Nachweis eines Antigens, z.B. HIV-p24, wird beispielsweise ein gegen dieses Antigen gerichteter Antikörper auf einer Testfläche immobilisiert. Als zum Nachweis dienender freier Rezeptor kann dann ein zweiter gegen das Antigen gerichteter, direkt oder indirekt markierter Antikörper, z.B. ein digoxigenylierter Anti-p24-Antikörper, verwendet werden. Zum Nachweis des entsprechenden, gegen das Antigen gerichteten Antikörpers, z.B. eines Anti-p24-Antikörpers, unter Verwendung einer Rücktitration wird ein mit dem Antikörper bindefähiges Antigen, z.B. p24, oder ein Fragment davon, auf einer weiteren Testfläche immobilisiert. Als Nachweisreagenz dient ebenfalls der zweite gegen das Antigen gerichtete markierte Antikörper, z.B. ein digoxigenylierter Anti-p24-Antikörper, welcher mit dem Analyten, das ist beispielsweise der in der Probe vorhandene natürliche Anti-p24-Antikörper um die Bindung an das immobilisierte Antigen kompetiert. So kann für den bevorzugten gleichzeitigen Nachweis eines p24-Antigens und des dagegen gerichteten Anti-p24-Antikörpers jeweils das gleiche Nachweisreagenz, beispielsweise ein digoxigenylierter Anti-p24-Antikörper verwendet werden.

Wenn der Nachweis des Antigens durch einen Sandwichtest und der parallele Nachweis des Antikörpers durch einen Brückentest oder einen indirekten Test erfolgt, müssen spezielle Testreagenzien verwendet werden, um eine wechselseitige Beeinflussung der Nachweisreagenzien auszuschließen. Bei einem indirekten Test zum Nachweis eines Antikörpers, z.B. eines Anti-p24-Antikörpers, wird beispielsweise ein Antigen, für das der nachzuweisende Antikörper spezifisch ist, z.B. p24, auf einer Testfläche immobilisiert. Zum Nachweis des Antikörpers wird dann ein markierter Antikörper, der zwar den nachzuweisenden Antikörper, aber nicht das immobilisierte Antigen erkennt, beispielsweise ein digoxigenylierter Anti-Human IgG-Antikörper verwendet. Bei der parallelen Antigenbestimmung im Sandwich-Format können auf der Nachweisseite ein oder mehrere Antikörper, bevorzugt monoklonale Antikörper, eingesetzt werden, deren Epitopbindestellen bekannt sind. Gleichzeitig können bei der AntikörperBestimmung im indirekten Testformat oder im Brückenformat keine nativen oder rekombinanten Antigene verwendet werden, die mit dem Nachweisantikörper bindefähigen Epitope enthalten, da sonst eine unerwünschte Reaktion stattfindet. Statt dessen müssen vorbestimmte, rekombinante Antigene oder Peptidantigene ohne diese Epitopbindestellen verwendet werden, an die der oder die eingesetzten Nachweisantikörper nicht bindefähig sind.

Durch die Multiepitopanalyse mit Arraystemen ist es möglich, eine Kombination von Antigen- und Antikörper-Nachweisen für ein bestimmtes Antigen und einen gegen dieses bestimmte Antigen gerichteten Antikörper durchzuführen. Diese Vorgehensweise ermöglicht es, die bei den im Stand der Technik bekannten Verfahren bestehende diagnostische Lücke zwischen dem ersten Auftreten eines Antigens und dem zeitlich versetzten Auftreten von Antikörpern zu schließen und eine Probe sehr früh als positiv bzw. negativ einzustufen. Üblicherweise werden Proben von Patienten genommen, wobei die Sensitivität eines Tests durch eine möglichst frühe Erkennung von positiven Proben bestimmt wird. Bei einer Infektion treten die verschiedenen Marker, die diese Infektionen anzeigen, wie beispielsweise Antigene oder gegen diese Antigene gerichteten Antikörper mit unterschiedlichem zeitlichen Verlauf auf.

Das Multiepitopverfahren mit einer Arrayanordnung ermöglicht zudem durch die räumlich getrennte Anordnung der einzelnen Testflächen eine spezifische Unterscheidung zwischen Antigen- und Antikörpertests. Der Vorteil des Verfahrens ist insbesondere bei HIV-Tests erkennbar. Ein bevorzugtes Beispiel für das Verfahren ist der gleichzeitige Nachweis eines HIV-Antigens und dagegen gerichteten Antikörper z.B. des p24-Antigens und des entsprechenden Anti-p24-Antikörpers. Bei einer HIV-Infektion treten zuerst p24-Antigene auf. Diese können mit einem Antigentest, nicht jedoch mit einem Antikörpertest nachgewiesen werden. Nach dem Auftreten der Antigene werden im Körper Antikörper gegen diese Antigene gebildet. Bei den herkömmlichen Kombitests ist es jedoch nicht möglich, den p24-Antigentest mit einem Anti-p24-Antikörpertest zu kombinieren, vielmehr findet eine Kombination des p24-Antigentests mit einem Anti-gp41-Antikörpertest statt. Da die Bildung von Anti-gp41-Antikörpern zeitlich jedoch nach der Bildung von Anti-p24-Antikörpern erfolgen kann, können im Zeitraum bis zur Bildung der Anti-gp41-Antikörper bei herkömmlichen Verfahren falsch negative Ergebnisse erhalten werden. Das erfindungsgemäße Verfahren ist demgegenüber sicherer, da auch Anti-p24-Antikörper, bestimmt werden können.

Bevorzugt wird die bindefähige Beschichtung der ersten Testfläche, in der das Antigen nachgewiesen werden soll, aus immobilisierten Antikörpern gebildet, die spezifisch für Epitope des nachzuweisenden Antigens sind. Aufgrund der bevorzugt verwendeten Arraystruktur ist es möglich, mehrere Antikörper, die für unterschiedliche Subtypen des nachzuweisenden Antigens spezifisch sind, in separaten Testflächen aufzubringen. Die Antikörper werden entsprechend dem zu analysierenden Antigen ausgewählt. Beim Screening auf eine virale Infektion werden bevorzugt Anti-HIV-I-Antikörper, Anti-HIV-II-Antikörper, Anti-HBV-Antikörper oder/und Anti-HCV-Antikörper getestet. Analog umfaßt die bindefähige Beschichtung der weiteren Testflächen, auf der ein Antikörper nachgewiesen werden soll, bevorzugt Antigene, die spezifisch für den nachzuweisenden Antikörper sind. Auch hier können grundsätzlich beliebige, auf den jeweiligen Test abgestimmte Antigene verwendet werden, bevorzugt werden Antigene oder Epitope davon aus HIV-I, HIV-II, HBV oder/und HCV verwendet.

Durch die Verwendung einer nichtporösen Festphase können besonders gute Ergebnisse mit dem Verfahren erhalten werden. Eine nichtporöse Festphase bietet insbesondere Vorteile beim Aufbringen der Testreagenzien, wobei ein definiertes Aufbringen ohne Ineinanderfließen der einzelnen Testflächen möglich ist. Weiterhin ist es bei Verwendung von nichtporösen Testphasen möglich, das Testformat zu miniaturisieren. Bei miniaturisierten Testformaten ist es möglich, eine Vielzahl von Testflächen auf eine einzige nichtporöse Festphase aufzubringen.

Der Nachweis der Bindung eines Antigens oder Antikörpers an die Testflächen wird bevorzugt unter Verwendung von markierten Antikörpern durchgeführt, die gegen den Analyten gerichtet sind. Beim Nachweis des Antigens im Sandwichformat wird dabei ein gegen dieses Antigen gerichteter markierter Antikörper verwendet. Der gleiche markierte Antikörper dient auch zum Nachweis des Analyten-Antikörpers beim kompetitiven Format, z.B. einer Rücktitration. Durch räumlich getrennte Auswertung der einzelnen Testflächen ist es somit möglich, mit einem einzigen Nachweisreagenz sowohl Antigen als auch den für dieses Antigen spezifischen Antikörper nachzuweisen, ohne daß sich die beiden Nachweisverfahren gegenseitig beeinträchtigen würden. Geeignete Markierungssubstanzen zur Markierung von Antikörpern sind dem Fachmann bekannt und umfassen z.B. fluoreszierende Gruppen, chemilumineszierende Gruppen, radioaktive Markierungen, Enzymmarkierungen, farbige Markierungen und Solpartikel. Bevorzugt ist die Verwendung eines universellen Nachweisreagenzes, insbesondere von fluoreszenzmarkierten Latexpartikeln, welche z.B. mit den Nachweisrzeptoren bindefähig ist.

Besonders gute Ergebnisse werden mit dem Verfahren erhalten, wenn die spezifisch bindefähigen Beschichtungen auf die einzelnen Testflächen separat aufgebracht werden. Dadurch ist es möglich, die Bindekapazität der einzelnen Testflächen optimal auszunutzen und optimal bindefähige Beschichtungen herzustellen. Gegebenenfalls können die bindefähigen Reagenzien durch Verdünnermoleküle verdünnt werden, um die Bindefähigkeit der Beschichtung weiter zu verbessern. Geeignete Verdünnermoleküle sind Moleküle, die nicht mit dem zu bestimmenden Analyten binden und die auch keine unspezifische Wechselwirkung oder Bindung zu anderen Probenbestandteilen zeigen, was zu falschpositiven Ergebnissen führen könnte (vgl. WO92/10757, EP 0 664 452 A2). Besonders bevorzugt wird die Beschichtung in den einzelnen Testflächen jeweils aus einem einzigen spezifisch bindefähigen Molekültyp gebildet. Unterschiedliche, mit dem Analyten bindefähige Reagenzien werden dabei in verschiedene Testspots aufgebracht. Auf diese Weise ist es möglich, die Sensitivität des erfindungsgemäßen Verfahrens weiter zu erhöhen.

Offenbart wird eine Festphase zur gleichzeitigen Bestimmung eines Antigens und eines spezifisch gegen dieses Antigen gerichteten Antikörpers, umfassend mindestens eine erste Testfläche und mindestens eine zweite Testfläche, welche dadurch gekennzeichnet ist, daß die erste Testfläche eine spezifisch mit einem Antigen bindefähige Beschichtung aufweist und die zweite Testfläche eine spezifisch mit einem gegen das Antigen gerichteten Antikörper bindefähigen Beschichtung aufweist, wobei die Beschichtungen homogen sind und jeweils nur einen einzigen Typ eines bindefähigen Reagenz enthalten. Die Beschichtungen sind gleichmäßig auf die Testflächen aufgebracht, das heißt, sie sind homogen. Neben dem bindefähigen Reagenz können die Testflächen inerte Verdünnermolküle umfassen, die weder mit dem nachzuweisenden Analyten noch mit anderen Probenbestandteilen Wechselwirkungen eingehen können.

Während grundsätzlich beliebige Trägermaterialien verwendet werden können, sind die Testflächen der erfindungsgemäßen Festphase bevorzugt auf einen nichtporösen Träger aufgebracht. Durch die Verwendung von nichtporösen Oberflächen ist insbesondere eine Miniaturisierung des Testformates und die gleichzeitige Bestimmung einer Vielzahl von Testflächen.

Die Festphase eignet sich insbesondere zur Verwendung in einem Immunoassay zum gleichzeitigen Nachweis eines Antigens und eines spezifisch gegen dieses Antigen gerichteten Antikörpers. Auf diese Weise ist es möglich, die Sensitivität und Zuverlässigkeit von Immuntests weiter zu verbessern.

Offenbart wird ein Testkit zur gleichzeitigen Bestimmung eines Antigens und eines spezifisch gegen dieses Antigen gerichteten Antikörpers, welches die Festphase sowie markierte Nachweisreagenzien zum Nachweis von an die Testflächen gebundenem Antigen und Antikörper umfasst. Geeignete Nachweisreagenzien sind beispielsweise markierte Antikörper, wobei die Markierung aus den oben genannten Gruppen ausgewählt sein kann.

Ein weiteres Problem bei den bisher verfügbaren Routentests besteht darin, daß alle für einen Test, beispielsweise einen HIV-Test, notwendigen Antigene und Antikörper gemischt werden und für das Nachweisverfahren eine für dieses Gemisch optimale Cut-Off-Grenze festgelegt wird. Durch die Verwendung einer gemeinsamen Cut-Off-Grenze für alle Parameter wird die Cut-Off-Grenze jedoch durch die unspezifische Bindung des schlechtesten Einsatzstoffes bestimmt und begrenzt. Offenbart wird deshalb ein Verfahren zum Nachweis eines Analyten in einer Probe, umfassend die Schritte:

(a) Bereitstellen einer Festphase, die einen Träger und mindestens zwei räumlich getrennte Testflächen umfaßt, wobei die Testflächen jeweils unterschiedliche, immobilisierte analytspezifische Rezeptoren enthalten,
(b) Inkontaktbringen der Probe mit der Festphase und mindestens einem freien analytspezifischen Rezeptor, der eine signalgebende Gruppe trägt oder mit einer signalgebenden Gruppe bindefähig ist, und
(c) Nachweisen des Vorhandenseins oder/und der Menge des Analyten durch Bestimmung der signalgebenden Gruppe auf den Testflächen, wobei ein Signa! oberhalb eines vorbestimmten Testflächen-spezifischen Grenzwertes als positiv und unterhalb eines vorbestimmten Testflächen-spezifischen Grenzwertes als negativ klassifiziert wird.

Durch die Verwendung von vorbestimmten Testflächen-spezifischen Grenzwerten für jede einzelne Testfläche ist es möglich, die Spezifität von Nachweisverfahren deutlich zu verbessern, während die Sensitivität unverändert hoch bleibt. Der Grenzwert oder Cut-Off-Wert wird mittels der Größen Signal der Probe, Hintergrund der Probe und Hintergrund einer Negativkontrolle bestimmt. Eine übliche Berechnung des Cut-Off-Wertes (COI) erfolgt beispielsweise nach der Formel:

$$COI = Signal_{Probe} - Untergrund_{Probe}/nxUntergrund_{Negativkontrolle}$$

Ein üblicher Wert für n beträgt beispielsweise 2. Der Faktor n - und damit der Cut-Off-Wert - kann für bestimmte Testflächen heraufgesetzt werden, bei denen falschpositive Proben beobachtet werden, wobei n eine Zahl zwischen 2 und 100, bevorzugt zwischen 2 und 10 sein kann. Bevorzugt werden die Grenzwerte jeweils individuell für eine Testfläche bestimmt. Dies bedeutet, daß für die unterschiedlichen Testflächen unterschiedliche Grenzwerte bzw. Cut-Off-Werte festgelegt werden, insbesondere werden die Grenzwerte für mindestens zwei Testflächen unterschiedlich festgelegt. Bevorzugte Ausführungsformen dieses Verfahrens machen von den oben beschriebenen Merkmalen Gebrauch.
Die Erfindung wird durch die folgenden Beispiele weiter erläutert.

**Beispiele**

### 1. Test auf Anti-HIV-Antikörper mit mehreren Antigen-spezifischen Testflächen mittels Microspot-Technologie

Microspot ist eine miniaturisierte ultrasensitive Technologie, die ideal zur gleichzeitigen Bestimmung von verschiedenen Parametern in einem einzigen Meßvorgang geeignet ist. Im Fall der Bestimmung von Anti-HIV-Antikörpern werden verschiedene HIV-Nachweisantigene in sogenannten "Arrays" jeweils einzeln mittels einer Inkjet-Methode auf eine Testfläche (Spot) auf einem Polystyrolträger aufgebracht. Bei der Testdurchführung werden 30 $\mu$l mit Probenpuffer im Verhältnis 1:1 verdünnte Probe auf den mit Testflächen versehenen Träger pipettiert und 20 Minuten unter Schütteln bei Raumtemperatur inkubiert. Nach Absaugen der Probe und Waschen mit Waschpuffer werden 30 $\mu$l Reagenzlösung 1, die eine Mischung aller Digoxigenin-markierten HIV-Antigene enthält, zugegeben und wiederum 20 Minuten unter Schütteln bei Raumtemperatur inkubiert. Nach Absaugen der Reagenzlösung 1 und Waschen mit Waschpuffer werden 30 $\mu$l Reagenzlösung 2 mit Nachweisreagenz zugegeben. Als universelles Nachweisreagenz dienen 100 nm große, fluoreszierende Latexpartikel, die kovalent mit einem Anti-Digoxigenin-Antikörper beschichtet sind.
Dieses Nachweisreagenz wird wiederum 20 Minuten unter Schütteln bei Raumtemperatur inkubiert, anschließend abgesaugt, gewaschen und trockengesaugt. Die Testflächen werden dann mit einem He-Ne-Laser mit 633 nm Wellenlänge bestrahlt und die Fluoreszenz bei 670 nm Wellenlänge mit einer CCD-Kamera vermessen.
Die Festphase enthält spezifische Testflächen mit folgenden immobilisierten Antigenen:

- rekombinantes p24 Polypeptid
- rekombinante Reverse Transkriptase (RT)
- gp41-Peptid 1
- gp41-Peptid 2

Als Probenpuffer wurde ein 50 mM Tris-Puffer pH 7,6 mit folgenden Zusätzen verwendet: 0,05% Tween 20, 0,5% Rinderserumalbumin (RSA), 0, 1 % Rinder-IgG, 0,01 % Methylisothiazolon, 3% Pepton.
Als Reagenzlösung 1 wurdederoben beschriebene Probenpufferverwendet, der folgende testspezifische Antigene enthält:

- Digoxigenin-markiertes, rekombinantes p24
- Digoxigenin-markierte, rekombinante Reverse Transkriptase
- Digoxigenin-markiertes gp41-Peptid 1
- Digoxigenin markiertes pg41-Peptid 2

Als Reagenzlösung 2 wurde ein 50 mM Tris-Puffer pH 8,0 mit folgenden Zusätzen verwendet: 0,05% Tween 20, 0,9% NaCl, 0,5% RSA, 0,1 % Naazid und 0,01% von fluoreszenzmarkierten, einem monoklonalen Anti-Digoxigenin-Antikörper beschichteten Latexpartikeln.

## 2. Vergleich eines Anti-HIV-Antikörper-Tests im Microspot-Format zu konventionellen Methoden

In diesem Versuch wurden sogenannte Serokonversionsproben vermessen. Diese Proben sind zeitlich aufeinanderfolgende Abnahmen verschiedener Personen, deren Serumbefund von HIV-negativ nach HIV-positiv konvertiert. Je sensitiver eine Testmethode ist, desto früher kann ein HIV-spezifisches Antikörper-Signal nachgewiesen werden. Die Proben wurden mit der Methode (Microspot) und vergleichend mit einer bekannten Methode (Enzymun® von Boehringer Mannheim) vermessen. Die dabei verwendeten, HIV-spezifischen Einsatzstoffe waren in beiden Testsystemen identisch, die sich daher vor allem nur durch die getrennte EinzelspotAnalyse unterscheiden. In der nachfolgenden Tabelle sind die Cut-off-Indices (Cut-off-Index = $Signal_{Probe}$-$Signal_{Untergrund}$/$2xSignal_{Negativkontrolle}$) der beiden Methoden aufgetragen und zusätzlich mit Westernblot-Daten verglichen:

| Serokonversions-Panel der Fa. BBI* | | Tag der Abnahme | p24 | RT | gp41-Peptid 1 | gp41-Peptid 2 | Western-Blot | Enzymun® |
|---|---|---|---|---|---|---|---|---|
| R | 2. Abnahme | 2 | 0.0 | 0.0 | 0.0 | 1.3 | negative | 0.5 |
| | 3. Abnahme | 7 | 22.2 | 0.0 | 0.0 | 2.4 | indifferent | 15.4 |
| | 4. Abnahme | 13 | 17.4 | 2.6 | 4.4 | 36.4 | positiv | 36.0 |
| AB | 1. Abnahme | 0 | 0.0 | 0.0 | 0.6 | 0.0 | negativ | 0.3 |
| | 2. Abnahme | 28 | 0.0 | 0.0 | 0.4 | 1.1 | negativ | 0.7 |
| | 3. Abnahme | 33 | 0.0 | 0.0 | 5.5 | 54.4 | negativ | 24.2 |
| | 4. Abnahme | 35 | 0.8 | 0.2 | 6.0 | 33.2 | positiv | 26.7 |
| | 5. Abnahme | 37 | 15.2 | 5.1 | 4.6 | 33.0 | positiv | 28.9 |
| AD | 5. Abnahme | 21 | 0.0 | 0.0 | 0.0 | 0.0 | negativ | 0.3 |
| | 6. Abnahme | 25 | 0.2 | 0.0 | 1.6 | 3.7 | positiv | 0.9 |
| | 7. Abnahme | 28 | 14.1 | 0.3 | 11.1 | 65.5 | positiv | 24.5 |
| AG | 3. Abnahme | 13 | 0.0 | 0.0 | 0.0 | 0.0 | negativ | 0.4 |
| | 4. Abnahme | 27 | 0.0 | 0.0 | 0.0 | 1.1 | negativ | 0.6 |
| | 5. Abnahme | 34 | 6.0 | 5.3 | 0.0 | 106.9 | positiv | 8.1 |
| | 6. Abnahme | 50 | 6.1 | 5.4 | 0.0 | 65.4 | positiv | 3.1 |
| | 7. Abnahme | 78 | 1.0 | 6.8 | 0.0 | 23.9 | positiv | 1.6 |
| | 8. Abnahme | 163 | 1.5 | 5.3 | 0.0 | 4.9 | positiv | 0.6 |
| | 9. Abnahme | 194 | 2.3 | 2.5 | 0.0 | 2.8 | positiv | 0.7 |
| AI | 1. Abnahme | 0 | 0.0 | 0.0 | 1.2 | 0.1 | indifferent | 0.8 |
| | 2. Abnahme | 7 | 0.6 | 0.5 | 54.7 | 44.9 | positiv | 30.2 |
| | 3. Abnahme | 11 | 1.1 | 0.7 | 18.8 | 22.5 | positiv | 30.2 |
| * Boston Biomedica Inc. | | | | | | | | |

Dieser Vergleich zeigt, daß durch die Aufteilung in Einzelspots mit jeweils optimalen Antigenkonzentrationen die Sensitivität im Vergleich zu bekannten Tests deutlich verbessert werden konnte. Von den 5 SerokonversionsPanels werden 7 Abnahmen früher positiv erkannt. Dies entspricht je nach Panel einer früheren Detektion der HIV-Infektion von 3 bis 7 Tagen. Auch im Vergleich zum Westernblot wurde eine deutliche Steigerung der Sensitivität mit 6 früher erkannten Abnahmen erreicht.

## 3. Vergleich einer kombinierten Bestimmung von HIV p24-Antigen sowie Anti-gp41- und Anti-RT-Antikörpern im Microspotformat zu konventionellen Methoden

Zur Evaluierung der Sensitivität wurden wiederum sogenannte Serokonversionsproben vermessen. Die Bestimmung erfolgte mit der Methode (Microspot) und die dabei erhaltenen Daten wurden mit den derzeit besten verfügbaren Anti-HIV-Tests (siehe Datenblätter der Hersteller von Serokonversionspanels, z.B. Firma BBI) bzw. mit dem Enzymun®-Kombinationstest von Boehringer Mannheim (kombinierte Bestimmung von p24-Antigen und Anti-HIV-Antikörpern) verglichen.

Für das Microspottestformat wurden folgende (wie in Beispiel 1 hergestellte) Testflächen (Einzelspots) verwendet:

- monoklonaler Anti-p24-Antikörper A zur Bestimmung des p24-Antigens von HIV Subtyp B
- monoklonaler Anti-p24-Antikörper B zur Bestimmung des p24-Antigens von HIV Subtyp B und O
- gp41-Peptid 1 zur Bestimmung von Antikörpern gegen gp41
- gp41-Peptid 2 zur Bestimmung von Antikörpern gegen gp41
- rekombinante Reverse Transkriptase (RT) zur Bestimmung von Antikörpern gegen RT

Die im Mikrospot-Test verwendeten HIV-spezifischen Einsatzstoffe waren vergleichbar mit den im Enzymun®-Test verwendeten Einsatzstoffen, so daß sich der Microspot-Test von Enzymun®-Methode vor allem nur durch die getrennte Einzelspotanalyse unterscheidet. In der nachfolgenden Tabelle sind die Cut-Off-Indices (Bestimmung siehe Beispiel 2) der beiden Methoden aufgeglistet und zusätzlich mit den bisher bekannten sensitivsten Anti-HIV-Tests verglichen.

| Serokonversions-Panel (Fa. BBI[xx]) | MAK <p24>A | MAK <p24>B | RT | gp41-Peptid 1 | pg41-Peptid 2 | sensi-tivster <HIV>-Test | Enzy-mun Kombi |
|---|---|---|---|---|---|---|---|
| Q 1. Abnahme | 0.0 | 0.0 | 0.0 | 0.0 | 0.3 | negativ | 0.30 |
| 2. Abnahme | 24.4 | 48 | 0.0 | 0.0 | 0.0 | negativ | 0.66 |
| 3. Abnahme | 246 | 435 | 0.0 | 0.0 | 0.0 | negativ | 3.47 |
| 4. Abnahme | nd | nd | nd | nd | nd | positiv | 2.30 |
| W 6. Abnahme | 0.1 | 0.0 | 0.1 | 0.0 | 0.1 | negativ | 0.30 |
| 7. Abnahme | 0.5 | 1.1 | 0.0 | 0.0 | 0.1 | negativ | 0.32 |
| 8. Abnahme | 5.8 | 14.1 | 0.1 | 0.0 | 0.1 | negativ | 0.41 |
| 9. Abnahme | 529 | 806 | 0.0 | 0.0 | 0.0 | positiv | 10.1 |
| Z 2. Abnahme | 0.0 | 0.0 | 0.0 | 0.0 | 0.0 | negativ | 0.31 |
| 3. Abnahme | 20 | 25.5 | 0.1 | 0.0 | 0.1 | negativ | 0.61 |
| 4. Abnahme | 226 | 262 | 0.0 | 0.0 | 0.0 | negativ | 2.96 |
| 5. Abnahme | 0.9 | 1.1 | 3.7 | 82.6 | 277 | positiv | 18.0 |
| AD 2. Abnahme | 0.0 | 0.0 | 0.0 | 0.0 | 0.1 | negativ | 0.30 |
| 3. Abnahme | 2.5 | 6.4 | 0.1 | 0.0 | 0.1 | negativ | 0.33 |
| 4. Abnahme | 96.8 | 200 | 0.0 | 0.0 | 0.0 | negativ | 1.5 |
| 5. Abnahme | 663 | 832 | 0.0 | 0.0 | 0.0 | positiv | >23.3 |
| 6. Abnahme | 549 | 709 | 0.6 | 2.7 | 2.8 | positiv | >23.3 |
| AF 3. Abnahme | 0.1 | 0.6 | 0.2 | 0.0 | 0.2 | negativ | 0.31 |
| 4. Abnahme | 0.4 | 1.7 | 0.0 | 0.0 | 0.02 | negativ | 0.30 |
| 5. Abnahme | 2.1 | 4.6 | 0.02 | 0.0 | 0.0 | negativ | 0.34 |
| 6. Abnahme | 31.2 | 61.6 | 0.1 | 2.9 | 204 | positiv | 18.0 |

xx BBI Boston Biomedica Inc.

Dieser Vergleich zeigt, daß die kombinierte Bestimmung von p24-Antigen und HIV-Antikörpern durch das Microspot-Testformat im Vergleich zu konventionellen Methoden deutlich verbessert werden kann. So ist der kombinierte Microspot-Test um ein Vielfaches sensitiver als der Enzymun®-Kombitest, bei dem alle Antigene und Antikörper in gemischter Form vorliegen. Bei den geprüften fünf Serokonversionspanels wurden im Vergleich zum sensitivsten Antikörpertest neun Abnahmen, im Vergleich zum Enzymun®-Kombitest immer noch sechs Abnahmen früher positiv detektiert.

**4. Kombinierte Bestimmung von p24-Antigen und Anti-p24-Antikörpern nach dem Rücktitrationsprinzip**

Zur kombinierten Bestimmung des p24-Antigens und von Antikörpern gegen p24 im selben Array-System wurde ein p24-Antigen-Test im Sandwichformat und ein Anti-p24-Antikörper-Test im Rücktitrationsformat durchgeführt.
Es wurden Arrays mit folgenden p24-spezifischen Reagenzien jeweils auf Einzelspots (siehe Beispiel 1) hergestellt:
(a) Panel mit p24-Antigen und Anti-p24-Antikörper-Test:

(i) p24-Antigen-Test:

Testfläche 1: monoklonaler Anti-p24-Antikörper A Fab'-Fragment, biotinyliert (100 $\mu$g/ml)

Testfläche 2: monoklonaler Anti-p24-Antikörper B Fab'-Fragment, biotinyliert (100 $\mu$g/ml)

(ii) Anti-p24-Test im Rücktitrationskonzept:

Testfläche 3: biotinyliertes p24-Antigen (0,3 $\mu$g/ml)

(b) Vergleichspanel mit Anti-p24-Antikörpertest im Brückenkonzept:

- biotinyliertes p24-Antigen (14 $\mu$g/ml)

Bei der Testdurchführung wurden zu jedem Panel 30 $\mu$l mit Probenpuffer im Verhältnis 1:1 verdünnte Probe pipettiert und 45 min unter Schütteln bei 37°C Inkubationstemperatur inkubiert. Nach Absaugen der Probe und Waschen mit Waschpuffer wurden 30 $\mu$l Reagenzlösung 1, die eine Mischung aller Digoxigenin-markierten HIV-Antigene und HIV-Antikörper enthält, zugegeben und 10 min unter Schütteln bei 37°C inkubiert. Folgende p24-spezifische Reagenzien wurden eingesetzt:

(a) Panel mit p24-Antigen- und Anti-p24-Antikörper-Test:

- monoklonaler Anti-p24-Antikörper D F(ab')$_2$-Fragment, digoxigenyliert (500 ng/ml)
- monoklonaler Anti-p24-Antikörper E F(ab')$_2$ Fragment, digoxigenyliert (500 ng/ml)

(b) Vergleichspanel mit Anti-p24-Antikörpertest im Brückenkonzept:

- digoxigenyliertes p24-Antigen (30 ng/ml)

Nach Absaugen der Reagenzlösung 1 und Waschen mit Waschpuffer wurden 30 $\mu$l Reagenzlösung 2 mit Nachweisreagenz (siehe Beispiel 1) zugegeben. Dieses Nachweisreagenz wurde 5 min unter Schütteln bei 37°C inkubiert, anschließend abgesaugt, gewaschen und getrocknet.

Das Testfeld wurde mit einem He-Ne-Laser der Wellenlänge 633 nm bestrahlt und die Fluoreszenz bei einer Wellenlänge von 670 nm mit einem konfokalen Laser-Scanner vermessen.

Mit beiden Panels wurden vergleichend 11 für HIV negative und 19 für HIV positive Proben vermessen: Die Cut-Off Indices (COI) für beide Testformate sind in der folgenden Tabelle angegeben.

| Probennummer | COI <p24> Rücktitration* | COI <p24> Brückenformat** |
|---|---|---|
| Negativkontrolle | 2412 Cts | 93 Cts |
| Positivkontrolle | 1276 Cts | 24651 Cts |
| Negativprobe 145 | 1.39 | 0.3 |
| Negativprobe 196 | 1.54 | 0.2 |
| Negativprobe 122 | 1.43 | 0.1 |
| Negativprobe 160 | 1.41 | 0.2 |
| Negativprobe 141 | 1.28 | 0.2 |
| Negativprobe 168 | 1.58 | 0.2 |
| Negativprobe 222 | 1.38 | 0.2 |
| Negativprobe 280 | 1.42 | 0.2 |
| Negativprobe 232 | 1.32 | 0.2 |
| Negativprobe 201 | 1.54 | 0.3 |

(fortgesetzt)

| Probennummer | COI <p24> Rücktitration* | COI <p24> Brückenformat** |
|---|---|---|
| Negativprobe 211 | 1.33 | 0.2 |
| Positivprobe 154 | 0.31 | 534 |
| Positivprobe 132 | 0.47 | 537 |
| Positivprobe 130 | 0.42 | 547 |
| Positivprobe 138 | 0.46 | 473 |
| Positivprobe 163 | 0.47 | 591 |
| Positivprobe 176 | 0.39 | 505 |
| Positivprobe 204 | 0.39 | 531 |
| Positivprobe 167 | 0.39 | 588 |
| Positivprobe 221 | 0.79 | 351 |
| Positivprobe 174 | 0.30 | 506 |
| Positivprobe 285 | 0.43 | 506 |
| Positivprobe 150 | 0.76 | 422 |
| Positivprobe 179 | 0.58 | 596 |
| Positivprobe 236 | 0.55 | 573 |
| Positivprobe 337 | 0.60 | 491 |
| Positivprobe 203 | 0.35 | 573 |
| Positivprobe 147 | 0.72 | 610 |
| Positivprobe 285 | 0.47 | 584 |
| Positivprobe 289 | 0.30 | 496 |
| *COI = $Signal_{Probe}$-$Signal_{Untergrund}$/$0,7 \times Signal_{Negativkontrolle}$; COI > 1,0 = negativ<br>** COI = $Signal_{Probe}$-$Signal_{Untergrund}$/$2x Signal_{Negativkontrolle}$; COI > 1,0 = positiv | | |

Sowohl alle negativen als auch alle positiven Proben konnten mit dem Rücktitrationsprinzip richtig detektiert werden. Durch die wechselwirkungsfreie Kombination von p24-Antigen-und Anti-p24-Antikörper-Tests können Serokonvertionsproben früher detektiert werden und die Sicherheit vor falsch negativen Detektionen zusätzlich erhöht werden.

**5. Verbesserung der Testspezifität durch Testflächen-spezifische Cut-Off-Berechnung**

In bisher verfügbaren Routinetests werden alle für die Bestimmung notwendigen Antigene und Antikörper vermischt und eine für dieses Gemisch optimale Cut-Off-Grenze festgelegt. Diese wird durch die unspezifische Bindung des "schlechtesten" Einsatzstoffes bestimmt. Durch die Microspot-Technologie kann man hingegen eine Testflächen-spezifische Cut-Off-Berechnung, die für jeden Einsatzstoff spezifisch ist, durchführen.

Bei identischer Berechnung des Cut-Off-Werts (COI = $Signal_{Probe}$-$Untergrund_{Probe}$/$2 x Untergrund_{Negativkontrolle}$) der einzelnen Testflächen konnte mit dem HIV-Kombinationstest (Beispiel 3) in 1264 Proben folgende Spezifität erreicht werden:

- p24-Antigen: 100%
- Anti-HIV-Antikörper-Test: 99,52% (sechs falsch positive Bestimmungen)

Da die falsch positiven Bestimmungen ausschließlich in den beiden Testflächen für gp41 Peptid 2 und Reverse Transkriptase auftraten, wurden die Cut-Off-Grenzen für diese Testflächen auf folgende Grenzwerte heraufgesetzt:

gp41-Peptid 2: $\quad COI = Signal_{Probe}\text{-}Untergrund_{Probe}/5 \times Untergrund_{Negativkontrolle}$

RT: $\quad COI = Signal_{Probe}\text{-}Untergrund_{Probe}/3 \times Untergrund_{Negativkontrolle}$

Auf diese Weise konnte die Spezifität des HIV-Tests von 99,52% auf 99,92% (nur noch eine einzige falsch positive Bestimmung) verbessert werden. Auch die Sensitivtät des Tests blieb unbeeinflusst, da der Cut-Off-Index des sensitiven p24-Antigentests nicht verändert wurde. So kann bei unverändert hoher Sensitivität eine deutliche Verbesserung der Spezifität erzielt werden.

**Patentansprüche**

1. Verfahren zur gleichzeitigen Bestimmung eines Antigens und eines spezifisch gegen dieses Antigen gerichteten Antikörpers in einer Probe, umfassend die Schritte:

   (a) Bereitstellen einer Festphase, auf der in einer ersten Testfläche ein mit dem zu bestimmenden Antigen bindefähiger immobilisierter Rezeptor und in einer räumlich davon getrennten zweiten Testfläche ein mit dem zu bestimmenden Antikörper bindefähiger immobilisierter Rezeptor aufgebracht ist,
   (b) Inkontaktbringen der Probe mit der Festphase und einem freien analytspezifischen Rezeptor, der eine signalgebende Gruppe trägt oder mit einer signalgebenden Gruppe bindefähig ist, und
   (c) Nachweisen des Vorhandenseins oder/und der Menge des Antigens und des Antikörpers durch Bestimmung der signalgebenden Gruppe auf der Festphase.

2. Verfahren nach Anspruch 1,
   **dadurch gekennzeichnet,**
   **daß** der Nachweis des Antigens unter Verwendung eines SandwichTests durchgeführt wird.

3. Verfahren nach einem der Ansprüche 1 oder 2,
   **dadurch gekennzeichnet,**
   **daß** der Nachweis des Antikörpers unter Verwendung eines Rücktitrationskonzepts durchgeführt wird.

4. Verfahren nach Anspruch 1 oder 2,
   **dadurch gekennzeichnet,**
   **daß** der Nachweis des Antikörpers unter Verwendung eines Brückenkonzepts durchgeführt wird.

5. Verfahren nach einem der Ansprüche 1 oder 2,
   **dadurch gekennzeichnet,**
   **daß** der Nachweis des Antikörpers unter Verwendung eines indirekten Testformats durchgeführt wird.

6. Verfahren nach einem der Ansprüche 1 bis 5,
   **dadurch gekennzeichnet,**
   **daß** die bindefähige Beschichtung der ersten Testfläche aus immobilisierten Antikörpern gebildet wird, die spezifisch für ein Epitop des nachzuweisenden Antigens sind.

7. Verfahren nach Anspruch 6,
   **dadurch gekennzeichnet,**
   **daß** Antikörper, die für unterschiedliche Subtypen des nachzuweisenden Antigens spezifisch sind, in separaten Testflächen aufgebracht werden.

8. Verfahren nach Anspruch 6 oder 7,
   **dadurch gekennzeichnet,**
   **daß** der Antikörper ausgewählt wird aus viralen Antikörpern, insbesondere Anti-HIV-I-Antikörpern, Anti-HIV-II-Antikörpern, Anti-HBV-Antikörpern und Anti-HCV-Antikörpern.

9. Verfahren nach einem der Ansprüche 1 bis 8,
**dadurch gekennzeichnet,**
**daß** die bindefähige Beschichtung der zweiten Testfläche aus Antigenen gebildet wird, die spezifisch für den nachzuweisenden Antikörper sind.

10. Verfahren nach Anspruch 9,
**dadurch gekennzeichnet,**
**daß** die Antigene ausgewählt werden aus der Gruppe bestehend aus HIV-I, HIV-II, HBV und HCV.

11. Verfahren nach einem der Ansprüche 1 bis 10,
**dadurch gekennzeichnet,**
**daß** es sich bei dem zu bestimmenden Antigen um HIV p24 und bei dem zu bestimmenden Antikörper um Anti-p24 handelt.

12. Verfahren nach einem der Ansprüche 1 bis 11,
**dadurch gekennzeichnet,**
**daß** eine nichtporöse Festphase verwendet wird.

13. Verfahren nach einem der Ansprüche 1 bis 12,
**dadurch gekennzeichnet,**
**daß** der Nachweis unter Verwendung von markierten Antikörpern, die gegen die Analyten gerichtet sind, durchgeführt wird.

14. Verfahren nach Anspruch 13,
**dadurch gekennzeichnet,**
**daß** die Markierung ausgewählt wird aus fluoreszierenden Gruppen, chemilumineszierenden Gruppen, radioaktiven Markierungen, Enzymmarkierungen, farbigen Markierungen und Solpartikeln.

15. Verfahren nach einem der Ansprüche 1 bis 14,
**dadurch gekennzeichnet,**
**daß** der Nachweis unter Verwendung eines universellen Nachweisreagenz, insbesondere markierten Latexpartikeln, durchgeführt wird.

16. Verfahren nach einem der Ansprüche 1 bis 15,
**dadurch gekennzeichnet,**
**daß** die Festphase durch direktes, separates Aufbringen der spezifisch bindefähigen Beschichtungen auf die einzelnen Testflächen hergestellt wird.

17. Verfahren nach einem der Ansprüche 1 bis 16,
**dadurch gekennzeichnet,**
**daß** die Beschichtung auf den Testflächen jeweils aus einem einzigen bindefähigen Molekültyp gebildet wird.

18. Verwendung einer Festphase gemäß eines Verfahrens der Ansprüche 1-17 zur gleichzeitigen Bestimmung eines Antigens und eines spezifisch gegen dieses Antigen gerichteten Antikörpers in einer Probe, umfassend mindestens eine erste Testfläche und mindestens eine zweite Testfläche,
**dadurch gekennzeichnet,**
**daß** die erste Testfläche eine spezifisch mit einem Antigen bindefähige Beschichtung aufweist und die zweite Testfläche eine spezifisch mit einem gegen das Antigen gerichteten Antikörper bindefähige Beschichtung aufweist.

19. Verwendung nach Anspruch 18,
**dadurch gekennzeichnet,**
**daß** die Beschichtungen homogen sind und jeweils nur einen einzigen Typ eines bindefähigen Reagenz enthalten.

20. Verwendung nach Anspruch 18 oder 19,
**dadurch gekennzeichnet,**
**daß** die Testflächen auf einen nichtporösen Träger aufgebracht sind.

21. Verwendung nach Anspruch 20,

**dadurch gekennzeichnet,**
**daß** der nichtporöse Träger aus Polystyrol gebildet ist.

**22.** Verwendung nach einem der Ansprüche 18 bis 21,
**dadurch gekennzeichnet,**
**daß** die einzelnen Testflächen einen Durchmesser von 0,01 bis 1 mm aufweisen.

**23.** Verwendung eines Testkits zur gleichzeitigen Bestimmung eines Antigens und eines spezifisch gegen dieses Antigen gerichteten Antikörpers umfassend eine Verwendung einer Festphase nach einem der Ansprüche 18 bis 22 sowie markierte Nachweisreagenzien.

**24.** Verwendung nach Anspruch 23,
**dadurch gekennzeichnet,**
**daß** das Teskit ein universelles Nachweisreagenz umfaßt.

**Claims**

**1.** Method for the simultaneous identification of an antigen and of an antibody directed specifically against this antigen, in a sample, comprising the steps of:

a) providing a solid phase to which is applied, in a first test area, an immobilised receptor capable of binding to the antigen to be identified and, in a second test area spatially separate therefrom, an immobilised receptor capable of binding to the antibody to be identified,
b) bringing the sample into contact with the solid phase and a free analyte-specific receptor, which carries a signalling group or is capable of binding to a signalling group, and
c) detecting the presence and/or the amount of antigen and antibody by identifying the signalling group on the solid phase.

**2.** Method according to claim 1, **characterised in that** the antigen is detected using a sandwich test.

**3.** Method according to either claim 1 or claim 2, **characterised in that** the antibody is detected using a back titration concept.

**4.** Method according to either claim 1 or claim 2, **characterised in that** the antibody is detected using a bridge concept.

**5.** Method according to either claim 1 or claim 2, **characterised in that** the antibody is detected using an indirect test format.

**6.** Method according to any of claims 1 to 5, **characterised in that** the coating, which is capable of binding, of the first test area is formed of immobilised antibodies that are specific for an epitope of the antigen to be detected.

**7.** Method according to claim 6, **characterised in that** antibodies that are specific for various sub-types of the antigen to be detected are applied in separate test areas.

**8.** Method according to either claim 6 or claim 7, **characterised in that** the antibody is selected from among viral antibodies, in particular anti-HIV-I antibodies, anti-HIV-II antibodies, anti-HBV antibodies and anti-HCV antibodies.

**9.** Method according to any of claims 1 to 8, **characterised in that** the coating, which is capable of binding, of the second test area is formed of antigens that are specific for the antibody to be detected.

**10.** Method according to claim 9, **characterised in that** the antigens are selected from the group consisting of HIV-I, HIV-II, HBV AND HCV.

**11.** Method according to any of claims 1 to 10, **characterised in that** the antigen to be identified is HIV p24 and the antibody to be identified is anti-p24.

**12.** Method according to any of claims 1 to 11, **characterised in that** a non-porous solid phase is used.

13. Method according to any of claims 1 to 12, **characterised in that** the detection is carried out using labelled antibodies which are directed against the analytes.

14. Method according to claim 13, **characterised in that** the label is selected from fluorescent groups, chemilumiscent groups, radioactive labels, enzyme labels, chromatic labels and sol particles.

15. Method according to any of claims 1 to 14, **characterised in that** the detection is carried out using a universal detection reagent, in particular labelled latex particles.

16. Method according to any of claims 1 to 15, **characterised in that** the solid phase is produced by directly and separately applying the coatings, which can bind in a specific manner, to the individual test areas.

17. Method according to any of claims 1 to 16, **characterised in that** the coating on the test areas is in each case formed of a single type of molecule that is capable of binding.

18. Use of a solid phase according to any method of claims 1 to 17 for the simultaneous identification of an antigen and of an antibody directed specifically against this antigen, in a sample, comprising at least one first test area and at least one second test area, **characterised in that** the first test area has a coating which can bind specifically to an antigen and the second test area has a coating which can bind to an antibody directed specifically against the antigen.

19. Use according to claim 18, **characterised in that** the coatings are homogenous and each contain just one type of reagent that is capable of binding.

20. Use according to either claim 18 or claim 19, **characterised in that** the test areas are applied to a non-porous substrate.

21. Use according to claim 20, **characterised in that** the non-porous substrate is formed of polystyrene.

22. Use according to any of claims 18 to 21, **characterised in that** the individual test areas have a diameter of from 0.01 mm to 1 mm.

23. Use of a testing kit for the simultaneous identification of an antigen and of an antibody directed specifically against this antigen, said testing kit comprising a use of a solid phase according to any of claims 18 to 22 and labelled detection reagents.

24. Use according to claim 23, **characterised in that** the testing kit comprises a universal detection reagent.

**Revendications**

1. Procédé de détection concomitante d'un antigène et d'un anticorps dirigé spécifiquement contre cet antigène dans un échantillon, comprenant les étapes de :

    (a) préparation d'une phase solide sur laquelle est appliqué dans une première surface d'essai, un récepteur immobilisé pouvant se lier à l'antigène à déterminer et dans une deuxième surface d'essai spécialement séparée de celle-ci, un récepteur immobilisé pouvant se lier à l'anticorps à déterminer,
    (b) mise en contact de l'échantillon avec la phase solide et avec un récepteur libre spécifique de l'analyte, qui porte un groupe de signalisation ou est capable de se lier à un groupe de signalisation, et
    (c) détection de la présence et/ou de la quantité de l'antigène et de l'anticorps par détermination du groupe de signalisation sur la phase solide.

2. Procédé selon la revendication 1, **caractérisé en ce que** la détection de l'antigène s'effectue en utilisant un test en sandwich.

3. Procédé selon l'une des revendications 1 ou 2, **caractérisé en ce que** la détection de l'anticorps s'effectue en utilisant un concept de rétrotitrage.

4. Procédé selon la revendication 1 ou 2, **caractérisé en ce que** la détection de l'anticorps s'effectue en utilisant un concept de pont.

5. Procédé selon l'une des revendications 1 ou 2, **caractérisé en ce que** la détection de l'anticorps s'effectue en utilisant un format de test indirect.

6. Procédé selon l'une des revendications 1 à 5, **caractérisé en ce que** le revêtement de la première surface d'essai pouvant se lier est formé d'anticorps immobilisés, qui sont spécifiques d'un épitope de l'antigène à détecter.

7. Procédé selon la revendication 6, **caractérisé en ce que** les anticorps qui sont spécifiques de différents sous-types de l'antigène à détecter sont appliqués dans des surfaces d'essai séparées.

8. Procédé selon la revendication 6 ou 7, **caractérisé en ce que** l'anticorps est choisi parmi les anticorps viraux, en particulier les anticorps anti-VIH I, les anticorps anti-VIH II, les anticorps anti-VHB et les anticorps anti-VHC.

9. Procédé selon l'une des revendications 1 à 8, **caractérisé en ce que** le revêtement de la deuxième surface d'essai pouvant se lier est formé d'antigènes qui sont spécifiques de l'anticorps à détecter.

10. Procédé selon la revendication 9, **caractérisé en ce que** les antigènes sont choisis dans le groupe comprenant VIH I, VIH II, VHB et VHC.

11. Procédé selon l'une des revendications 1 à 10, **caractérisé en ce que** l'antigène à déterminer est VIH p24 et l'anticorps à déterminer est l'anti-p24.

12. Procédé selon l'une des revendications 1 à 11, **caractérisé en ce que** l'on utilise une phase solide non poreuse.

13. Procédé selon l'une des revendications 1 à 12, **caractérisé en ce que** la détection est réalisée en utilisant des anticorps marqués, qui sont dirigés contre les analytes.

14. Procédé selon la revendication 13, **caractérisé en ce que** le marquage est choisi parmi les groupes fluorescents, les groupes chimioluminescents, les marquages radioactifs, les marquages enzymatiques, les marquages colorés et les particules en solution.

15. Procédé selon l'une des revendications 1 à 14, **caractérisé en ce que** la détection est réalisée en utilisant un réactif de détection universel, en particulier des particules de latex marquées.

16. Procédé selon l'une des revendications 1 à 15, **caractérisé en ce que** la phase solide est produite par application directe, séparée, des revêtements pouvant se lier spécifiquement, sur les surfaces d'essai individuelles.

17. Procédé selon l'une des revendications 1 à 16, **caractérisé en ce que** le revêtement sur les surfaces d'essai est formé respectivement à partir d'un type de molécule unique pouvant se lier.

18. Utilisation d'une phase solide selon un procédé selon les revendications 1 à 17, pour la détermination concomitante d'un antigène et d'un anticorps dirigé spécifiquement contre cet antigène dans un échantillon, comprenant au moins une première surface d'essai et au moins une deuxième surface d'essai, **caractérisée en ce que** la première surface d'essai présente un revêtement pouvant de lier spécifiquement à un antigène et la deuxième surface d'essai présente un revêtement pouvant se lier spécifiquement à un anticorps dirigé contre l'antigène.

19. Utilisation selon la revendication 18, **caractérisée en ce que** les revêtements sont homogènes et contiennent respectivement, seulement un type unique d'un réactif pouvant se lier.

20. Utilisation selon la revendication 18 ou 19, **caractérisée en ce que** les surfaces d'essai sont appliquées sur un support non poreux.

21. Utilisation selon la revendication 20, **caractérisée en ce que** le support non poreux est formé de polystyrène.

22. Utilisation selon l'une des revendications 18 à 21, **caractérisée en ce que** les surfaces d'essai individuelles présentent un diamètre de 0,01 à 1 mm.

**23.** Utilisation d'un kit d'essai pour la détermination concomitante d'un antigène et d'un anticorps dirigé spécifiquement contre cet antigène, comprenant une utilisation d'une phase solide selon l'une des revendications 18 à 22 et de réactifs de détection marqués.

**24.** Utilisation selon la revendication 23, **caractérisée en ce que** le kit d'essai comprend un réactif de détection universel.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0461462 A1
- EP 0627625 A1
- EP 0445423 A2
- US 5432099 A
- US 5516635 A
- US 5126276 A
- WO 9210757 A
- EP 0664452 A2
- US PS5627026 A

**In der Beschreibung aufgeführte Nicht-Patentliteratur**

- **EKINS ; CHU.** *Clin. Chem.,* 1995, vol. 37, 1955-1967